# EUROPEAN PATENT SPECIFICATION

(11) **EP 2 923 697 B1**
(45) Date of publication and mention of the grant of the patent: **17.01.2018**
(21) Application number: 14161927.0
(22) Date of filing: 27.03.2014
(51) Int. Cl.: A61K 31/045, A61K 36/06, A61P 31/00

(54) **Extract and novel isolated compound from Aspergillus terreus for treatment of mouth microbial infections**
Extrakt und neuartige isolierte Verbindung aus Aspergillus terreus zur Behandlung von mikrobieller Mundinfektionen
Extrait et nouveau composé isolé à partir d'aspergillus terreus pour le traitement d'infections microbiennes buccales

(43) Date of publication of application: 30.09.2015
(73) Proprietor: King Saud University, 11421 Riyadh (SA)
(72) Inventor: Awaad, Amani Shafeek, 11421 Riyadh (SA); Al-Othman, Monerah Rashed, 11333 Riyadh (SA); Zain, Mohamed El-Desouky Mohamed, 11333 Riyadh (SA); El-Meligy, Reham Moustafa, 11421 Riyadh (SA); Al-Mudhayyif, Hind Ahmed, 11333 Riyadh (SA)
(74) Representative: Goddar, Heinz J.

(56) References cited:
- US-A- 4 420 491
- AMANI S. AWAAD ET AL: "New Antifungal Compounds from Aspergillus terreus Isolated from Desert Soil", PHYTOTHERAPY RESEARCH, vol. 26, no. 12, 16 December 2012 (2012-12-16), pages 1872-1877, XP055137763, ISSN: 0951-418X, DOI: 10.1002/ptr.4668
- PUGLIESE A ET AL: "Effects of Aspergillus terreus extract on herpes simplex 1 virus replication", JOURNAL OF CHEMOTHERAPY, vol. 7, no. 1, 1995, pages 33-37, XP9179923, ISSN: 1120-009X
- DATABASE WPI Week 200777 Thomson Scientific, London, GB; AN 2007-817784 XP002729338, & CN 1 966 667 A (YUNNAN MICROORGANISM RES INST) 23 May 2007 (2007-05-23)
- DATABASE WPI Week 198606 Thomson Scientific, London, GB; AN 1986-039714 XP002729339, & JP S60 260570 A (SS PHARMACEUTICAL KK) 23 December 1985 (1985-12-23)

## Description

The present invention relates to an extract and an isolated compound from *Aspergillus terreus* for use in the treatment of mouth microbial infections of bacteria. The fungal kingdom includes many species with unique and unusual biochemical pathways. The products of these pathways include important pharmaceuticals such as penicillin, cyclosporin and statins; potent poisons including aflatoxins and trichothecenes; and some Janus-faced metabolites that are both toxic and pharmaceutically useful such as the ergot alkaloids. All of these natural products, along with many other low-molecular weight fungal metabolites, are classified together as secondary metabolites, see Keller et al., Nat. Rev. Microbiol., 3, 937-947 (2005). The fungal secondary metabolites have a wide range of chemical structure and biological activities. They are derived from many different intermediates by special enzymatic pathways encoded by specific genus. The fungal secondary metabolites, or biochemical indicators of fungal development, are of intense interest to humankind due to their pharmaceutical and/or toxic properties.
Soil-derived fungi have been recognized as a prolific source of biologically active metabolites, see Wang et al., J. Nat. Prod., 73, 942-948 (2010). Soil fungal community diversity and functioning remains poor relative to that of soil bacterial communities, and it is not uncommon for articles that purport to review aspects of 'soil microbial ecology' to consider only bacteria, see for example Hattori et al., Antonie Van Leeuwenhoek, 71, 21-28 (1997). Fungi perform important services related to water dynamics, nutrient cycling and disease suppression. Along with bacteria, fungi are important as decomposers in the soil food web, converting hard to digest organic material into usable forms.
The biological activities of fungal extracts in naturally occurring compounds are comprehensively mentioned by many researchers, see for example Zain et al., J. Appl. Sci. Res., 5, 1582-1591 (2009); Phytopharmacology, 2, 106-113 (2012) and Awaad et al., Phytother. Res. 26, 1872-1877 (2012).
Awaad *et al.* describe two butyrolactone derivatives and one carboxylic acid methyl ester derivative which were identified from a strain of *Aspergillus terreus* Thom (Trichocomaceae) isolated from desert soil. The antifungal activities of extracts as well as of both intra- and extracellular metabolites of *Aspergillus terreus* grown on yeast extract sucrose (YES) and malt extract media (MEA) were determined.
Further state of the art describe the usage of tissue conditioner in combination with antifungal agents or plant oils on *Candida albicans,* see Falah-Tafti et al., Dent. Res. J., 7, 18-22 (2010) and Catalán et al., Oral. Surg. Oral. Med. Oral. Pathol. Oral. Radiol. Endod., 105, 327-332 (2008). Tissue conditioner is a treatment material which is used to re-establish tone and.health to irritated oral soft tissue, usually applied to the edentulous alveolar ridge.
Most tissue conditioners have more or less supporting role in adhesion and growth of *Candida* which subsequently cause denture stomatitis in the oral cavity of denture wearers. It was believed that a tissue conditioner with antimicrobial activity could be a great advantage for patients with a high risk of denture stomatitis. Numerous researches have investigated the combination of certain additives and antifungals to tissue conditioner as a drug delivery method for controlling microbial attachment and colonization, see for example Geerts et al., J. Oral. Rehabl., 35, 664-669 (2008).
It is an object of the present invention to provide a novel and effective antimicrobial agent for treating mouth infection of bacteria which overcomes the drawbacks of the prior art. In particular, an antimicrobial agent shall be provided having improved activity against fungal and bacteria, but without being hepatotoxic or having a negative impact on liver and kidney functions.
This object is achieved by an extract from *Aspergillus terreus* for use in the treatment of mouth microbial infections of bacteria. The extract is combined with a tissue conditioner. Preferably, the extract is an alcoholic extract, more preferably an ethanolic extract.
Furthermore, it is preferred that the extract is used for the treatment of Gram-negative bacteria, Gram-positive bacteria preferably *Lactobacillus acidophilus, Streptococcus gordonii* and *Streptococcus mutan.*
By the term "extract" within the present application is meant an extract which contains all the compounds which have been extracted with a respective solvent from the fungal broth.
The object is further achieved by a compund of Fomula I for use in the treatment of mouth microbial infections of bacteria. 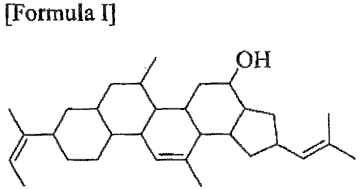 The compound is combined with tissue conditioner. Finally, the object is achieved by a pharmaceutical composition comprising the inventive extract and/or the compound of Formula I, in combination with tissue conditioner. The pharmaceutical composition is for treating mouth microbial infections. of bacteria Surprisingly, it was found that an alcoholic extract of *Aspergillus terreus* and a compound isolated from *Aspergillus terreus,* in combination with tissue conditioner can be used as antimicrobial agents for the treatment of microbial infections of fungi and bacteria. The antimicrobial agents exhibit similar or even higher microbial activity compared to drugs known from the prior art. Further, it was found that the alcoholic extract and isolated compound of *Aspergillus terreus,* preferably, in combination with tissue conditioner can be used for the treatment of mouth microbial infections without having any side effects such as hepatotoxicity or a negative impact on liver and kidney functions.

Secondary metabolites produced by *Aspergillus terreus* showed a great activity against *Candida albicans, Lactobacillus acidophilus, Streptococcus gordonii,* and S. *mutan.* However, the MICs of the isolated compound were lower as the ones of extract and isolated compound combined with tissue conditioner. The alcoholic extract of *Aspergillus terreus* and isolated compound in combination with tissue conditioner inhibited the growth of *Candida albicans* when used in concentrations of 500 µg/ml and 7.81 µg/ml, respectively; *Lactobacillus acidophilus* when used in concentrations of 250 µg/ml and 7.81 µg/ml, respectively; *Streptococcus gordonii* when used in concentrations of 1000 µg/ml and 62.50 µg/ml, respectively; and *S. mutans* when used in concentrations of 1000 µg/ml and 125 µg/ml, respectively. The oral dosing of the extract and the isolated compound did not show any significant effect on the activity of aspirate aminotransferase (AST), alanine aminotransferase (ALT) and the levels of blood urea and serum creatinine.

The tissue conditioner used within the present invention is preferably Coe-comfort™ tissue conditioner which belongs to the category of acrylic resins, phthalic acids tissue conditioning (dental).

The term "pharmaceutical composition", as used herein, is intended to comprise at least one pharmaceutical active extract in combination with a tissue conditioner and/or at least the isolated compound and/or corresponding salts thereof, preferably; in combination with the tissue conditioner.

The pharmaceutical composition can be, for example, in a liquid form, e.g. a solution, syrup, elixir, emulsion and suspension, or in a solid form, e.g. a capsule, caplet, tablet, pill, powder, and suppository. Granules, semi-solid forms and gel caps are also considered. In case that the pharmaceutical composition is a liquid or a powder, dosage unit optionally is to be measured, e.g. in the dosage unit of a teaspoon. In addition to the extract or the isolated compound in combination with the tissue conditioner, the pharmaceutical composition can comprise, for example, flavoring agents, sweeteners, dyes, stabilizers, diluents, suspending agents, granulating agents, lubricants, binders and disintegrating agents. A tablet, for example, can be coated. All of the formulations mentioned can be intended for immediate-release, timed release and sustained release.

All components of the pharmaceutical composition have to be pharmaceutically acceptable. The term "pharmaceutically acceptable" means at least non-toxic. The therapeutically active component should be preferably be present in the above-mentioned pharmaceutical composition in a concentration of about 0.1 to 99.5% by weight, preferably of about 0.5 to 95% by weight of the total mixture.

The above-mentioned pharmaceutical composition can further contain other pharmaceutical active compounds in addition to the active extract and/or compound according to the invention.

Additional features and advantages of the present invention will become apparent in the following detailed description on the basis of the examples.

### Examples

### Materials and methods

### Fungal isolation and growth conditions

The fungal strain was isolated from desert soil samples collected from Riyadh, Kingdom of Saudi Arabia. The malt extract agar (MEA) medium (malt extract, 20 g; peptone, 1 g; dextrose, 20 g; agar, 20 g; distilled water, 1 1) was used for isolation, cultivation, and identification of the fungal isolate and test organisms. Nutrient Agar (NA) (Peptone, 5 g; beef extract, 3 g; sodium chloride, 3g, Agar, 20 g; distilled water, 1 1) was used for cultivation of bacterial test organisms. For production of secondary metabolites, yeast extract sucrose agar (YES) (yeast extract, 20 g; sucrose, 150 g; agar, 20 g; distilled waster, 11) was used.

### Test organisms

Mouth microbial representatives including unicellular fungi; *Candida albicans* (RCMB 05382), *Candida glabrata* (RCMBA 05386), *Candida parapsilosis* (RCMBA 06002), *Candida tropicalis* (RCMB 05384), Gram-positive bacteria; *Lactobacillus acidophilus* (RCMBA 4267), *Streptococcus gordonii* (RCMBA 2004), *Streptococcus mutans* (RCMBA 4131), and Gram-negative bacteria; *Pseudomonas aeruginosa* (RCMBA 1002) were obtained from the Regional Center for Mycology and Biotechnology (RCMB), Al-Azhar University, Cairo, Egypt, as test organisms.

### Extraction

The mat of *Aspergillus terreus,* grown on YES (700 g), was extracted by percolation in ethanol (4 1) at room temperature for two days and filtered. The residue was re-percolated again and the process repeated four times. The combiried ethanol extracts were concentrated under reduced pressure at a temperature not exceeding 35°C to yield a dry extract (120 g).

### Isolation of active compound

The dried ethanolic extract for intracellular growth of *Aspergillus terreus* was fractionated using a silica gel (450 g) column and eluted gradually with hexane/ether/chloroform. 120 fractions were collected (60 ml each) and those which were chromatographically similar were combined to produce 5 main fractions. These were concentrated under reduced pressure to yield 3.34, 1.32, 1.6 and 5.21 g, respectively. Each fraction was reapplied multiple times on the top of other columns packed with a silica gel and eluted gradually with hexane/petroleum ether. Hereby, compound of Formula I was obtained as white crystals (750 mg). Identification was realized by using different instrumental analysis (¹H-NMR, ¹³C-NMR, mass and melting points).

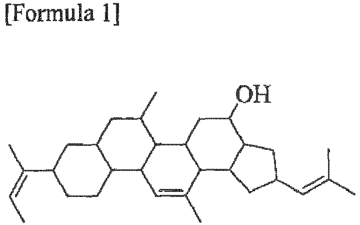

R_{F} = 0.56 in system (hexane : ether (60: 40 v/v)). M.p: 513-514°C. Molecular Weight was 436 g/mol and its molecular formula was found to be C₃₁H₄₈O. The compound was identifed as:
9-((Z)-but-2-en-2-yl)-2,3,3a,4,5,5a;5b,6,7,7a,8,9,10,11,11a,11b,13a,13b-octadecahydro-6,13-dimethyl-2-(2-methylprop-1-enyl)-1H-cyclopenta[a]chrysen-4-ol. The compound was found to be a novel compound.

### Pharmacological studies

### Antimicrobial activity

Antimicrobial activity was determined by the well diffusion method. Petri plates contained 20 ml of Nutrient (for bacteria) or Malt extract (for fungi). Agar medium were seeded with 1-3 day cultures of microbial inoculums (1-2 X 10⁷ CFU/ml). Wells (6 mm in diameter) were cut off into agar and 50 µl of extract and isolated compound were tested in a concentration of 100 mg/ml and incubated at 37°C for 24-48h. The assessment of antimicrobial activity was based on measurement of the diameter of the inhibition zone formed around the well.

### Minimum inhibitory concentration (MIC) determination

The minimum inhibitory concentration (MIC) was determined by micro-dilution method using serially diluted (2 folds) extract and isolated compound. MIC of the extract was determined by dilution of extract and isolated compound of concentrations of 0.0-1000 µg/ml. Equal volume of each extract and nutrient broth were mixed in a test tube. Specifically 0.1 ml of standardized inoculum (1-2 X 10⁷ CFU/ml) was added in each tube. The tubes were incubated at 37°C for 24-48 h. Two control tubes containing the growth medium, saline and the inoculum were maintained for each test batch. The lowest concentration (highest dilution) of the extract that produced no visible microbial growth (no turbidity) when compared with the control tubes were regarded as MIC.

### In-vitro Activity of extract of Aspergillus terreus and isolated compound in combination with tissue conditioners on Candida albicans

The Coe-Comfort tissue conditioner was mixed according to the proportions indicated by the manufacturers. In detail, Coe-comfort™ tissue conditioner is formed by dispensing 5 ml of Coe-comfort liquid into a disposable mixing cup. The red measure level is filled with about 6 g of Coe-comfort powder and is then slowly added to the liquid. The obtained mixture is blended for 30 seconds and setted aside for approximately one minute until it flows sluggishly from a spatula. Using less liquid gives a thicker, faster setting mix and vice versa. Doses of total alcoholic extract and isolated compound; 1000, 500, 250, 125, 62.5, 31.25, 15.63, 7.81, 3.9, and 1.95 µg/ml were added to the conditioner. The extract and isolated compound/conditioner mixture was homogenized in a sterile glass beaker for 30 seconds. Immediately afterwards, the conditioning powder was added and mixed for 40 seconds and then poured onto a Petri plate. Once the mixture solidified, the surface was covered with 4 ml of Sabouraud agar, and after 30 minutes, inoculated with 20 µl of an appropriate dilution of *Candida.* The plates were incubated at 37°C for 24 hours and then the CFUs were counted. The experiment was performed in triplicate. For the conditioner Coe-Comfort, the control was unmodified conditioner.

Activity of amphotericin B and chlorhexidine in combination with tissue conditioners in dosses of 31.25, 15.63, 7.81, 3.9, 1.95, 0.98, 0.49, 0.24, 0.12, and 0.06 µg/ml were determined.

### Acute toxicity (LD₅₀) test

The oral median lethal dose (LD₅₀) of the ethanolic extract of the investigated fungi was determined. Swiss albino mice (both sex 26-30 g) in groups of six, received one of 500, 1000, 2000, or 5000 mg/kg doses of the tested extract. Control animals were received the vehicle and kept under the same conditions. Signs of acute toxicity and number of deaths per dose within 24 h were recorded.

### Sub-chronic Toxicity:

Male Wister rats (150-180 g) were divided into 3 equal groups each of 10 rats. Rats of the 1st group received the vehicle in a dose of 5 mL/kg and left as normal control. Rats of the 2nd and 3rd groups were administered the ethanolic extract of the investigated fungi (500 mg/kg) and the isolated compound (50 mg/kg). All medications were administered orally daily for 15 consecutive days. Animals were maintained under identical conditions with food and water ad libitum for the entire period with close observation. At the end of the experimental period, blood samples were collected from the orbital plexus of rats, 6 hr after the last dose. Samples were left to clot at room temperature for 20 min. The obtained sera were collected and used to determine the activity of AST and ALT. Levels of urea and creatinine were also estimated.

### Results and Discussion

A fungal isolate was isolated from soil sample collected from desert of Riyadh, KSA and identified *Aspergillus terreus.* The antimicrobial activity of *A. terreus* grown on yeast extract sucrose broth and isolated compound showed a great activity against the investigated test organisms (Table 1). The alcoholic extract and isolated compound showed a great activity against all the investigated Gram-negative and Gram-positive bacteria and against 3, out of 4, *Candida* species (Table 1).

The activity of the isolated compound was better than the total extract and it was more or less similar to the standard antibiotics. However, the best activities (23.9 ± 0.37; MIC 0.24 µg/ml and 22.4 ± 0.58; MIC 0.49 µg/ml) were obtained by the isolated compound against *Lactobacillus acidophilus* and *Candida tropicalis,* respectively (Tables 1,2).

The activity of alcoholic extract from *Aspergillus terreus* and isolated compound in combination with Coe-comfort tissue conditioner on *Candida albicans* revealed total growth inhibition of C. *albicans* when used in concentrations of 500 µg/ml of extract and 7.81 µg/ml of isolated compound (Table 3). However, 7.81 µg/ml of amphotericin B was effective in inhibiting C. *albicans* growth when used in combination with Coe-comfort tissue conditioner (Table 3). The activity of the isolated compound on C. *albicans* was almost similar to the activity of amphotericin B.

**Table 1. Antimicrobial activity of alcoholic extract and isolated compound of Aspergillus terreus against clinically isolated mouth microbial infectious. Mean zone of inhibitions of 5mg/ml of sample around well (6 mm) were determined in mm ± Standard deviation.**

| **Test organism** | **Diameter of inhibition zone (mm)** | | |
|---|---|---|---|
| | **Extract** | **Isolated compound** | **Standard antibiotic** |
| **Unicellular fungi** | | | Amphotericin B |
| *Candida albicans* | 14.2 ± 0.58 | 19.3 ± 0.63 | 21.3± 0.14 |
| *Candida glabrata* | 00.0 | 00.0 | 20.4± 0.87 |
| *Candida tropicalis* | 17.1 ± 0.44 | 22.4 ± 0.58 | 24.6± 0.27 |
| *Candida parapsilosis* | 16.2 ± 0.25 | 21.3 ± 0.58 | 23.2 ± 0.53 |

| **Bacteria** | | | Chlorhexidine |
|---|---|---|---|
| *Pseudomonas aeruginosa* | 15.8 ± 0.58 | 18.2± 0.63 | 20.1 ± 0.19 |
| *Lactobacillus acidophilus* | 20.4 ± 0.25 | 23.9 ± 0.37 | 26.2± 0.42 |
| *Streptococcus gordonii* | 17.8 ± 0.44 | 20.3 ± 0.72 | 22.1± 0.51 |
| *Streptococcus mutans* | 18.3 ± 0.37 | 22.1± 0.72 | 23.5 ± 0.34 |

**Table 2. The minimum inhibitory concentration (MIC) of alcoholic extract and isolated compound of Asperpillus terreus against clinically isolated mouth microbial infectious.**

| **Test organism** | **Minimum Inhibitory Concentration (µg/ml)** | | |
|---|---|---|---|
| | **Extract** | **Isolated compound** | **Standard antibiotic** |
| **Unicellular fungi** | | | Amphotericin B |
| *Candida albicans* | 125.00 | 007.81 | 001.95 |
| *Candida glabrata* | 000.00 | 000.00 | 001.95 |
| *Candida tropicalis* | 031.25 | 000.49 | 000.12 |
| *Candida parapsilosis* | 062.50 | 001.95 | 000.49 |

| **Bacteria** | | | Chlorhexidine |
|---|---|---|---|
| *Pseudomonas aeruginosa* | 062.50 | 015.63 | 003.90 |
| *Lactobacillus acidophilus* | 003.90 | 000.24 | 000.06 |
| *Streptococcus gordonii* | 015.63 | 0003.9 | 000.98 |
| *Streptococcus mutans* | 015.63 | 000.98 | 000.49 |

On the other hand, the activity of alcoholic extract of *Aspergillus terreus* and isolated compound in combination with Coe-comfort tissue conditioner on *Lactobacillus acidophilus* showed total growth inhibition when used in concentrations of 250 µg/ml of extract and 7.81 µg/ml of isolated compound (Table 4). However, 15.63 µg/ml of chlorhexidine inhibited growth of *L. acidophilus* when used in combination with Coe-comfort tissue conditioner (Table 4). The activity of the isolated compound on *Lactobacillus acidophilus* was better than the activity of standard antibiotic, chlorhexidine (Table 4).

**Table 3. Activity of Coe-Comfort mixed with alcoholic extract of Aspergillus terreus and isolated compound on Candida albicans.**

| **Concentration (µg/ml)** | **No. colony-forming units (CPU)** | | |
|---|---|---|---|
| | **Extract** | **Isolated compound** | **Amphotericin B** |
| **000.00 (Control)** | 9.2 x 10⁴ | 9.2 x 10⁴ | 9.2 x 10⁴ |
| **000.24** | ND | 9.1 x 10⁴ | ND |
| **000.49** | ND | 5.2 x 10⁴ | 9.1 x 10⁴ |
| **000.98** | ND | 6.3 x 10³ | 8.9 x 10⁴ |
| **001.95** | ND | 1.0 x 10³ | 9.3 x 10³ |
| **003.90** | ND | 2.3 x 10² | 8.1 x 10² |
| **007.81** | 9.1 x 10⁴ | 00.00 | 00.00 |
| **015.63** | 8.9 x 10⁴ | 00.00 | 00.00 |
| **031.25** | 9.2 x 10³ | 00.00 | 00.00 |
| **062.50** | 4.2 x 10³ | 00.00 | 00.00 |
| **125.00** | 2.1 x 10³ | ND | ND |
| **250.00** | 2.6 x 10² | ND | ND |
| **500.00** | 00.00 | ND | ND |
| **1000.0** | 00.00 | ND | ND |

| | | | |
|---|---|---|---|
| ND, not determined. | | | |

**Table 4. Activity of Coe-Comfort mixed with alcoholic extract of Aspergillus terreus and isolated compound on Lactobacillus acidophilus.**

| **Concentration (µg/ml)** | **No. colony-forming units (CFU)** | | |
|---|---|---|---|
| | **Extract** | **Isolated compound** | **Chlorhexidine** |
| **000.00 (Control)** | 1.3 x 10⁶ | 1.3 x 10⁶ | 1.3 x 10⁶ |
| **000.24** | ND | 8.2 x 10⁵ | 6.4 x 10⁵ |
| **000.49** | ND | 9.7 x 10⁴ | 2.3 x 10⁵ |
| **000.98** | ND | 4.8 x 10⁴ | 3.1 x 10⁴ |
| **001.95** | ND | 3.4 x 10³ | 5.4 x 10³ |
| **003.90** | ND | 4.1 x10² | 1.9 x 10³ |
| **007.81** | 8.2 x 10⁵ | 00.00 | 7.9 x 10² |
| **015.63** | 4.1x10⁵ | 00.00 | 00.00 |
| **031.25** | 6.2 x 10⁴ | 00.00 | 00.00 |
| **062.50** | 4.4 X 10³ | 00.00 | 00.00 |
| **125.00** | 3.1 X 10² | ND | ND |
| **250.00** | 00.00 | ND | ND |
| **500.00** | 00.00 | ND | ND |
| **1000.0** | 00.00 | ND | ND |

| | | | |
|---|---|---|---|
| ND, not determined. | | | |

The activities of alcoholic extract of *Aspergillus terreus* and isolated compound in combination with Coe-comfort tissue conditioner on *Streptococcus gordonii* and *S. mutans* were determined (Tables 5 and 6). The total extract of *A. terreus* showed growth inhibition on *Streptococcus gordonii* and *S. mutans* when used in concentrations of 1000 µg/ml of extract. However, the isolated compound showed total inhibition on *Streptococcus gordonii* and *S. mutans* at concentrations of 62.5 and 125 µg/ml, respectively (Tables 5 and 6). However, chlorhexidine inhibited growth of *Streptococcus gordonii* and *S. mutans* at concentrations of 3.9 and 15.63 µg/ml, respectively.

## Claims

1. Extract from *Aspergillus terreus* combined with a tissue conditioner for use in the treatment of mouth microbial infections of bacteria.

2. Extract according to claim 1, wherein the extract is an alcoholic extract, preferably ethanolic extract.

3. Extract according to claim 1 or 2, wherein the extract is used for the treatment of Gram-negative bacteria, and/or Gram-positive bacteria, preferably *Lactobacillus acidophilus, Streptococcus gordonii* and *Streptococcus mutan.*

4. Compound of Formula I combined with a tissue conditioner for use in the treatment of mouth microbial infections of bacteria.

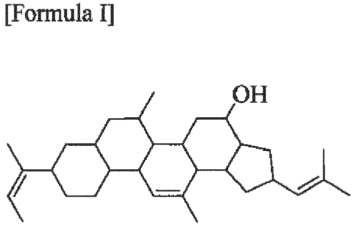

5. Pharmaceutical composition for use in the treatment of mouth microbial infections of bacteria comprising an extract according to claims 1 to 3 and/or a compound of Formula I according to claim 4.

## Patentansprüche

1. Extrakt von *Aspergillus terreus* kombiniert mit einem Gewebekonditionierer zur Verwendung bei der Behandlung von mikrobakteriellen Infektionen des Mundraums durch Bakterien.

2. Extrakt nach Anspruch 1, wobei das Extrakt ein alkoholisches Extrakt, vorzugsweise ein ethanolisches Extrakt, ist.

3. Extrakt nach Anspruch 1 oder 2, wobei das Extrakt zur Behandlung von Gram-Negativ-Bakterien und/oder Gram-Positiv-Bakterien verwendet wird, vorzugsweise *Lactobacillus acidophilus, Streptococcus gordonii* und *Streptococcus mutan.*

4. Verbindung nach Formel I zusammen mit einem Gewebekonditionierer zur Verwendung bei der Behandlung von mikrobakteriellen Infektionen des Mundraums durch Bakterien.

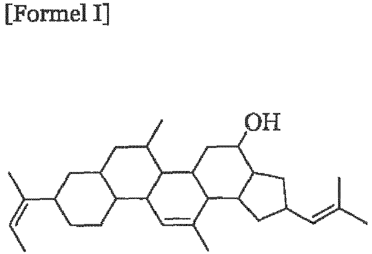

5. Pharmazeutische Zusammensetzung zur Verwendung bei der Behandlung von mikrobakteriellen Infektionen des Mundraums durch Bakterien umfassend ein Extrakt nach einem der Ansprüche 1 bis 3 und/oder eine Verbindung der Formel I nach Anspruch 4.

## Revendications

1. Extrait d'*Aspargillus terreus* combiné à un conditionneur tissulaire à utiliser dans le traitement d'infections microbiennes buccales de bactéries.

2. Extrait selon la revendication 1, où l'extrait est un extrait alcoolique, de préférence un extrait éthanolique.

3. Extrait selon la revendication 1 ou 2, où l'extrait est utilisé dans le traitement de bactéries à Gram-négatif, et/ou de bactéries à Gram-positif, de préférence *Lactobacillus acidophilus, Streptococcus gordonii* et *Streptococcus mutans.*

4. Composé de la Formule I combiné à un conditionneur tissulaire à utiliser dans le traitement d'infections microbiennes buccales de bactéries.

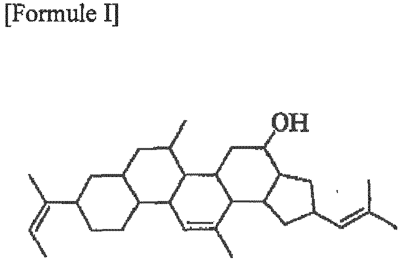

5. Composition pharmaceutique à utiliser dans le traitement d'infections microbiennes buccales de bactéries comprenant un extrait selon les revendications 1 à 3 et/ou un composé de la Formule I selon la revendication 4.
